# EUROPÄISCHE PATENTANMELDUNG

(11) **EP 0 594 887 A1**
(43) Veröffentlichungstag der Anmeldung: **04.05.1994**
(21) Anmeldenummer: 92118564.1
(22) Anmeldetag: 29.10.1992
(51) Int. Cl.: G01N 33/44, G01N 27/66

(54) **Verfahren zur Erkennung von Kunststoffen sowie deren anschliessende Sortierung**

(71) Anmelder: Van Der Haegen, Hans Mueller Prof. Dr., D-25764 Wesselburener Koog (DE)
(72) Erfinder: Van Der Haegen, Hans Mueller Prof. Dr., D-25764 Wesselburener Koog (DE)

(57) **Zusammenfassung**

Diese Erfindung befaßt sich mit einer Anordnung zur Identifikation von Kunststoffen unter gleichzeitiger Erkennung von Kunststoffen mit flammenhemmenden oder sonstigen Zuschlagstoffen auf Halogen-, Phosphat- oder Metallbasis bestehend aus einer Einheit zur thermischen Zersetzung der Probe, einer Leitung zur Heranführung der zersetzten Probe an die Analyseeinheit, einer Analyseeinheit, bestehend aus einem Massenspektrometer und einer unter Spannung stehenden speziellen Diode, sowie einer Steuer- und Auswerteeinheit, in der im Vergleich der in der Analyseeinheit aufgenommenen mit eingespeicherten Werten die Identität des untersuchten Stoffes bestimmt und ein der Identifizierung des Stoffes entsprechendes Signal registriert, ausgegeben oder z.B. zur Steuerung einer Sortierstrecke weitergeleitet oder in anderer Form verarbeitet wird. Die erwähnte unter Spannung stehende spezielle Diode kann auch allein als Analyseeinheit Verwendung finden, wenn nur in den Kunststoffen enthaltene Halogene oder Zuschlagstoffe detektiert werden sollen. Es ist weiterhin möglich, diese Diode auch mit anderen als der massenspektrometrischen Detektionsmethode zu kombinieren.

## Beschreibung

Zur Sortierung von Kunststoffen aller Art z.B. zum Zwecke ihrer Wiederverwertung bedarf es eines Verfahrens zu deren Identifizierung, daß sowohl schnell, als auch zuverlässig arbeitet und durch einfache Probenzuführungsmöglichkeit auch in Sortierstrecken einsetzbar ist, wobei das System dazu geeignet sein sollte, aufgrund des Identifikationsergebnisses automatisch auf die Steuerung der Sortierung Einfluß nehmen zu können. Insbesondere ist es von Nöten, solche Kunststoffe gesondert zu erkennen, die z.B. in Form halogenierter Stoffe Zusätze zur Flammhemmung enthalten, welche bei der weiteren Verarbeitung an der Bildung von unerwünschten Beiprodukten beteiligt sein könnten oder sich in anderer Weise bei der weiteren Verwendung der Stoffe negativ auswirken könnten. Eine weitere Notwendigkeit besteht in der Erkennung sogenannter Biopolymere, die die Weiterverarbeitung durch anderes Mischungsverhalten stören und durch anderes Biodegradationsverhalten die Beständigkeit der Produkte ungünstig beeinflussen könnten. Eine Anordnung, die die oben genannten Forderungen erfüllt, wird in dem Verfahren dieser Erfindung vorgestellt. Das vorgestellte Analysesystem eignet sich zur Erkennung und Zuordnung von Kunststoffen aus dem Hausmüll, aber auch aus anderen Bereichen, wie des Automobilbaus, der Elektronik und Elektrotechnik und ist insbesondere in der Lage, Flammschutzmittel enthaltende Kunststoffe sicher zu detektieren und eine entsprechende Sortierung anzusteuern.

Heute fallen in der Bundesrepublik Deutschland jährlich etwa 1,8 Millionen Tonnen Kunststoffabfälle an. Die Wiederverwertung dieser Kunststoffe wird zwar vom Gesetzgeber gefordert, einzelne Ansätze hierzu wurden unternommen, aufgrund der großen Heterogenität des Ausgangsmaterials konnten bisher jedoch nur wenige befriedigende Ergebnisse erzielt werden. Wegen der begrenzten Verträglichkeit von unterschiedlichen Kunststoffen untereinander führt deren Vermischung in der Regel zu einer drastischen Eigenschaftsverschlechterung. Aufgrund dieser begrenzten Mischbarkeit und wegen der zu erwartenden schwankenden Qualitäten solcher Mischungen infolge schwankender Anteile der Einzelkomponenten bieten z.B. gemischte Kunststoffabfälle nur sehr begrenzte Möglichkeiten der Wiederverwertung.

Hinzu kommt bei der thermischen Prozessierung der Kunststoffabfälle die Möglichkeit der Bildung von die Gesundheit schädigenden Beiprodukten unter Beteiligung von flammhemmenden Additives, wie sie vor allem in Kunststoffen aus den Bereichen der Elektronik und Elektrotechnik, aber auch im Automobilbau Verwendung fanden, bzw. zumindest im internationalen Rahmen auch heute noch finden.

Eine Alternative kann eine sortenreine Trennung der Kunststoffabfälle und Ausschluß der flammgehemmten Kunststoffe bieten, nach der Fraktionen mit weit enger gefaßten Spezifikationen zu verarbeiten wären.

Die bisherigen Verfahren zur sortenreinen Trennung von Kunststoffen haben bislang jedoch nur geringen Eingang in die Praxis finden können. Hier sind Verfahren der Kennzeichnung, Händische Sortierung, Trennung aufgrund unterschiedlicher Dichte, Infra-rot-Detektion zu nennen.

Wenn auch die Gründe hierfür vielfältiger Natur sein mögen, so befriedigen die herkömmlichen Methoden insgesamt nicht bei der Erkennung von halogenierten und anderen Zusatzstoffen der Kunststoffe, die oft aus Flammschutzgründen den Kunststoffen zugeschlagen wurden.

Diese Aufgabe wird erfindungsgemäß gelöst durch eine Anordnung zur Identifikation vor Kunststoffen unter gleichzeitiger Erkennung von Kunststoffen mit flammenhemmenden oder sonstigen Zuschlagstoffen auf Halogen-, Phosphat- oder Metallbasis bestehend aus einer Einheit zur thermischen Zersetzung der Probe, einer Leitung zur Heranführung der zersetzten Probe an die Analyseeinheit, einer Analyseeinheit, bestehend aus einer Massenspektrometer und einer unter Spannung stehenden speziellen Diode, sowie einer Steuer- und Auswerteeinheit, in der im Vergleich der in der Analyseeinheit aufgenommenen mit eingespeicherten Werten die Identität des untersuchten Stoffes bestimmt und ein der Identifizierung des Stoffes entsprechendes Signal registriert, ausgegeben oder z.B. zur Steuerung einer Sortierstrecke weitergeleitet oder in anderer Form verarbeitet wird. Die erwähnte unter Spannung stehende spezielle Diode kann auch allein als Analyseeinheit Verwendung finden, wenn nur in den Kunststoffen enthaltene Halogene oder Zuschlagstoffe detektiert werden sollen. Es ist weiterhin möglich, diese Diode auch mit anderen als der massenspektrometrischen Detektionsmethode zu kombinieren. Die zu detektierenden Stoffe bewirken in der Diode eine Veränderung des Ionenstromes, der als Meßgröße zur Detektion herangezogen wird.

Das vorgestellte Verfahren nutzt die Eigenschaft der Kunststoffe, beim Erhitzen einer thermischen Abbauprozeß zu unterliegen und dabei spezifische Zersetzungsprodukte zu emittieren. Der Transport der Gasproben zur Analyseeinheit kann zum Schutz vor weiterer Veränderung in Schutzgasatmosphäre erfolgen. Für die Entwicklung zur Detektion ausreichender Gasvolumina genügt die kurzzeitige Erwärmung einer kleinen Probe. Dieses kann sowohl berührend, als auch ohne Berührung mittels Wärme-, Licht- oder anderer Strahlung erfolgen.

Anordnungen nach den erfindungsgemäßen Verfahren können sowohl als von Hand zu bedienen ausgeführt sein, als auch in automatisch arbeitende Sortieranlagen integriert werden, sie sind unempfindlich und universell einsetzbar, gekennzeichnet durch kurze Detektionszeiten und in der Lage, auch leicht verschmutzte und lackierte Kunststoffe ansprechen.

Die Möglichkeit, zusätzlich zur Kunststoffart Aussage über dessen flammhemmende Formulierung treffen zu können, stellt ein Novum dar.

Weitere Einzelheiten der Erfindung sind aus den nachfolgenden Beispielen zu ersehen.

### Beispiele:

### Beispiel 1:

In einer Sortieranlage befindet sich hinter einer Vereinzelung ein über ein elektro-optisches System gesteuerter Gelenkarm, an dem ein mit Schutzgas beaufschlagter Plasmabrenner und eine Schnüffelleitung befestigt sind. Der Plasmabrenner wird zu jedem Probestück bis auf einen kurzen Abstand abgesenkt und durch die Hitze ein kleines Stück des Probestücks thermisch zersetzt. Das entstehende Gas wird mittels einer nachgeschalteten Pumpe in die Schnüffelleitung gezogen und ein Teilstrom in ein Quadrupol-Massenspektrometer abgesaugt. Ein weiterer Teilstrom gelangt in eine Zelle, die vermittels einer Vakuumpumpe ständig unter Unterdruck gehalten wird. In dieser Zelle befinden sich eine elektrisch beheizte Platinkathode und eine sie mit geringen Abstand umgebende Anode mit einer angelegten Spannung von z.B. 150 V. Die Zelle wird abwechselnd mit Probegas befüllt und mit Frischluft gespült. Der Ionenstrom zwischen den Elektroden dient als Meßgröße zur Detektion. Seine Änderung wird erfaßt und in einem Computer mit gespeicherten Werten verglichen. Das Massenspektrometer besteht aus einem Rezipienten, einem HF-Analysator, einem Dosierventil, einer Vakuumvorpumpe, einer Turbomolekularpumpe und Steuerungskomponenten. In dem Massenspektrometer werden in kurzen Abständen ausgewählte Massen aufgenommen und das Ergebnis in dem Computer ebenfalls mit gespeicherten Daten verglichen. Aus der Kombination der prozessierten Daten aus beiden Aufnahmeverfahren ermittelt der Rechner den Sortierweg für das Probestück und gibt entsprechende Signale an die anschließend vom Probestück durchlaufene Sortierstrecke.

### Beispiel 2:

Zur Detektion von Halogenen und flammhemmenden Zuschlagstoffen in Kunststoffen ist die Diodenzelle in einem Handgerät untergebracht. Das Handgerät trägt weiterhin eine mit der Spitze einer elektrischen Lötpistole vergleichbare Einheit zur thermischen Zersetzung eines kleinen Teils des Probenstückes. Über eine kurze Schnüffelleitung wird das Analysengas mittels einer außerhalb des Handgerätes gelegenen Vakuumpumpe in die Meßzelle gesaugt, die danach, über Magnetventile gesteuert, wieder mit Frischluft gespült wird. In einer ebenfalls im Handgerät untergebrachten Auswerteeinheit wird die Änderung des Ionenstroms in der Meßzelle prozessiert, mit gespeicherten Daten verglichen und die Detektion der fraglichen Stoffe optisch und akustisch zur Anzeige gebracht.

## Patentansprüche

1. Verfahren zur Identifikation von Kunststoffen und Erkennung sogenannter Biopolymere dergestalt, daß eine Probe des zu untersuchenden Kunststoffes thermisch zersetzt, die Bruchstücke in einem Massenspektrometer analysiert und aus dem Vergleich spezieller Merkmale der aufgenommenen mit gespeicherten Daten die Identität des untersuchten Stoffes bestimmt wird.

2. Anordnung zur Identifikation von Kunststoffen und Erkennung sogenannter Biopolymere nach dem Verfahren nach Anspruch 1 bestehend aus einer Einheit zur thermischen Zersetzung der Probe, einer Leitung zur Heranführung der Bruchstücke an die Analyseneinheit, einem Massenspektrometer, dem mindestens ein Teil des Gasstroms zugeleitet wird, als Analyseneinheit und einer Steuer- und Verrechnungseinheit, in der im Vergleich der in der Analyseneinheit aufgenommenen mit eingespeicherten Werten die Identität des untersuchten Stoffes bestimmt und ein der Identifizierung des Stoffes entsprechenden Signals mindestens registriert oder weitergeleitet oder verarbeitet wird.

3. Verfahren zur Detektion von Halogenen, Phosphaten und Metallen in Kunststoffkomponenten und Zuschlagstoffen zu Kunststoffen auf Halogen-, Phosphat- oder Metallbasis dergestalt, daß eine thermisch zersetzte Probe des Kunststoffs einer unter Spannung stehenden speziellen Diode zugeleitet wird und eine bestimmte Veränderung des Ionenstroms zwischen den Elektronen als Indikationsparameter für das Vorhandensein der nachzuweisenden Stoffe genutzt wird.

4. Anordnung zur Detektion von halogenierten Kunststoffkomponenten und Zuschlagstoffen zu Kunststoffen auf Halogen-, Phosphat- oder Metallbasis nach dem Verfahren nach Anspruch 2 bestehend aus einer einer Einheit zur thermischen Zersetzung der Probe, einer Leitung zur Heranführung der Zersetzungsprodukte an die Analyseneinheit, einer Analyseneinheit, in der mindestens ein Teil des Gasstroms einer unter Spannung stehenden speziellen Diode zugeleitet wird und einer elektronischen Steuer- und Verrechnungseinheit, in der im Vergleich der in der Analyseneinheit aufgenommenen Werten der Veränderungen des Ionenstroms zwischen den Elektronen mit eingespeicherten Werten das Vorkommen der genannten Stoffe in dem untersuchten Stoffes bestimmt und ein der Detektion entsprechenden Signals mindestens registriert oder weitergeleitet oder verarbeitet wird.

5. Verfahren zur Identifikation von Kunststoffen und Erkennung sogenannter Biopolymere unter gleichzeitiger Erkennung von Kunststoffen mit flammenhemmenden oder sonstigen Zuschlagstoffen auf Halogen-, Phosphat- oder Metallbasis dergestalt, daß die Verfahren nach Anspruch 1 und Anspruch 3 kombiniert werden und die Verarbeitung der gewonnenen Daten die Ergebnisse aus beiden Verfahren im Gesamtergebnis miteinbezieht.

6. Anordnung zur Identifikation von Kunststoffen und Erkennung sogenannter Biopolymere unter gleichzeitiger Erkennung von Kunststoffen mit flammenhemmenden oder sonstigen Zuschlagstoffen auf Halogen-, Phosphat- oder Metallbasis nach dem Verfahren nach Anspruch 5 bestehend aus einer einer Einheit zur thermischen Zersetzung der Probe, einer Leitung zur Heranführung der zersetzen Probe an die Analyseneinheit, einem Massenspektrometer und und eine unter Spannung stehenden spezielle Diode als Analyseneinheit und einer Steuer- und Verrechnungseinheit, in der im Vergleich der in der Analyseneinheit aufgenommenen mit eingespeicherten Werten die Identität des untersuchten Stoffes bestimmt und ein der Identifizierung des Stoffes entsprechenden Signals mindestens registriert oder weitergeleitet oder verarbeitet wird.

7. Verfahren zur Identifikation von Kunststoffen unter gleichzeitiger Erkennung von Kunststoffen mit flammenhemmenden oder sonstigen Zuschlagstoffen auf Halogen-, Phosphat- oder Metallbasis dergestalt, daß das Verfahren nach Anspruch 3 mit einem anderen Verfahren zur Kunststofferkennung als dem Verfahren nach Anspruch 1 Kombiniert wird.
